(19) Europäisches Patentamt
European Patent Office
Office européen des brevets

(11) EP 4 664 465 A1

(12) EUROPEAN PATENT APPLICATION

(43) Date of publication:
**17.12.2025 Bulletin 2025/51**

(21) Application number: **25182758.0**

(22) Date of filing: **13.06.2025**

(51) International Patent Classification (IPC):
***G16C 60/00*** *(2019.01)*

(52) Cooperative Patent Classification (CPC):
**G16C 60/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **14.06.2024 IT 202400013690**

(71) Applicant: **Inrete Distribuzione Energia S.p.A.
40127 Bologna (IT)**

(72) Inventors:
- **COLUCCIO, Luigi
40139 BOLOGNA (IT)**
- **NANNETTI, Emanuele
40055 CASTENASO (BOLOGNA) (IT)**
- **VALENTE, Alessandro
44123 FERRARA (IT)**

(74) Representative: **Milli, Simone
Bugnion S.p.A.
Via di Corticella, 87
40128 Bologna (IT)**

(54) **METHOD AND APPARATUS FOR ESTIMATING THE CONCENTRATION OF AN ODORANT, COMPUTER PRODUCT TO CARRY OUT THE METHOD AND DISTRIBUTION SYSTEM**

(57) A method implemented by means of a processor (5) is disclosed for estimating the concentration of an odorant in a gas pipeline (2) using a mathematical function that takes into account the concentration of the odorant flowing in the gas pipeline and the fact that a part of the odorant is absorbed on the walls of the gas pipeline. The method comprises subdividing the pipe (2) in a plurality of virtual portions (2A, 2B) of the pipe (2); making calculations relating to the virtual pipe portions by means of a mathematical function to calculate the concentration of the odorant in the two different pipe portions at a first time instant; and using the same mathematical function to calculate the concentration of the odorant in the second pipe portion (2B) at a second time instant.

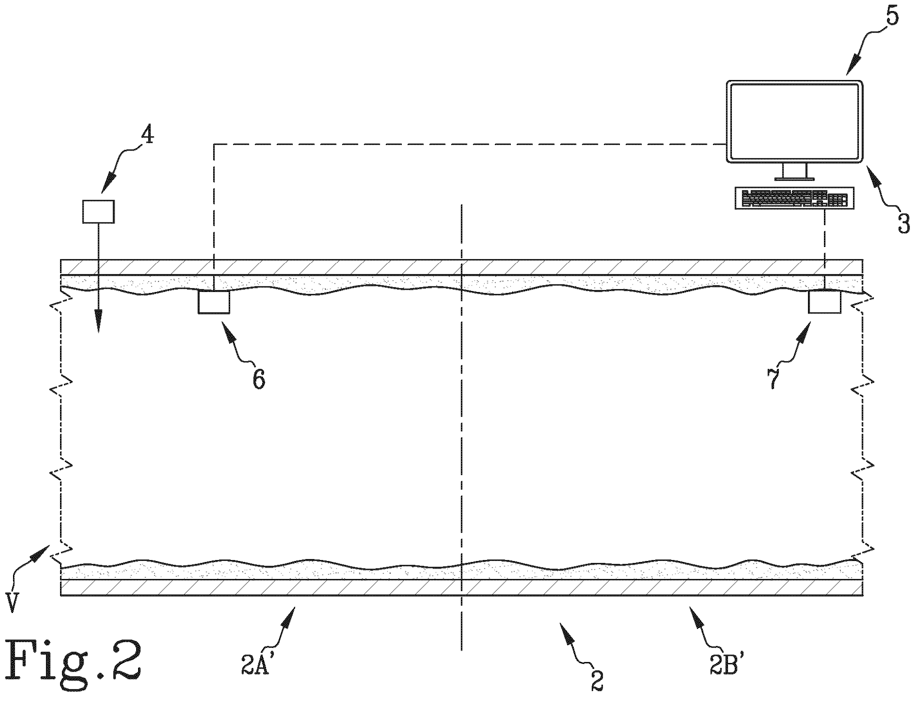

Fig.2

EP 4 664 465 A1

## Description

The present invention relates to a method for estimating the concentration of an odorant, a computer product to carry out the method, an estimation apparatus and a distribution system.

The natural gas for household use or similar has no characteristic odour sufficient to be detected before potential dangers for toxicity and explosive conditions occur.

The gas, odourless per se, is odorized by the introduction of a substance, called odorant, which gives the gas a characteristic odour in order to guarantee the user safety.

Such odorization is carried out by the gas distribution companies.

Among the many kinds of odorants used, the ones constituted by tetrahydrothiophene (THT) are particularly widespread.

So, the gas distribution networks comprise odorization systems to introduce the odorant so that the gas is sufficiently odorized, when used by domestic users or similar.

Disadvantageously, the odorant tends to adhere to the walls of pipes through which the gas is distributed.

In fact, the inner surfaces of the pipes, through which the gas is distributed, are covered by iron oxides that cause odorant reversible absorption.

This phenomenon causes significant odorant losses, since the quantity of odorant introduced in the odorization system is significantly lower than the one provided when the gas is supplied to domestic users.

This could reduce user safety, in case the odorant contained in the gas is not sufficient to be detected in case of loss.

Such problem is particularly felt with variations of gas concentrations in input and of gas flow rates in the pipes.

At the same time, dissolving an excessive quantity of odorant in the gas would lead to waste the odorant itself, incurring also in high costs.

So, there is the need to solve the above-mentioned problems by providing a method for estimating the concentration of an odorant in order to guarantee that the quantity of odorant provided in the gas, when supplied to domestic users, is sufficient to guarantee user safety.

Aim of the present invention is to provide a method for estimating the concentration of an odorant, a computer product to carry out the method, an estimation apparatus and a distribution system which solve at least the above-mentioned need, thus guaranteeing safety of users using the gas in their homes.

Other features and advantages of the present invention will be clearer from the following description, which is indicative, and so not limitative, of a preferred embodiment of the invention, as it is shown in the appended drawings, in which:

- figure 1 shows a schematic view of a processor to execute a computer product according to the present description,
- figure 2 shows a schematic view of a gas distribution system according to the present description,
- figure 3 shows a graph representing over time, as a comparison, a concentration of odorant calculated by means of a method for estimating the concentration of an odorant in a gas according to the present description and a measured concentration of odorant.

Object of the present description is a method, implemented by means of a processor 5, for estimating the concentration of an odorant in a gas flowing in a pipe 2.

Preferably, but non limitingly, the odorant referred is THT.

Preferably, but not limitingly, the gas flowing in the pipe is methane.

The method comprises a step of subdividing the pipe 2 in a plurality of virtual portions 2A, 2B of the pipe 2, said plurality of virtual portions 2A, 2B comprising at least a first virtual portion 2A and a second virtual portion 2B of the pipe 2.

It is to be observed that, according to an aspect, such virtual portions 2A, 2B of the pipe 2 represent real portions of a pipe.

The virtual portion 2A is to be considered as the upstream portion to the virtual portion 2B, according to a conveying direction of the gas in the pipe 2.

The virtual portion 2A, 2B represents a real portion, i.e. physical, of a gas conveying pipe, i.e. that preferably defines a model of a physical portion.

The virtual portions 2A, 2B represent a respective real portion 2A', 2B'.

In other words, it is called virtual portion since the method is processed by means of a processor 5.

In an example, the pipe 2 is thought to be a linear, cylindrical and with constant diameter pipe, without side connections.

The method comprises a step of receiving, i.e. setting (and storing) a first parameter $Ca0_a$, representing at a predetermined initial time $t_0$, the odorant concentration in gaseous phase defining a mobile portion, inside the first virtual portion 2A. In other words, the term odorant mobile portion indicates a quantity of the odorant in gaseous phase contained inside the respective virtual portion.

The method comprises a step of receiving, i.e. setting (and storing) a second parameter $Ca0_b$, representing at a predetermined initial time $t_0$, the odorant concentration in gaseous phase defining a mobile portion, inside the second virtual portion 2B.

The method comprises a step of receiving, i.e. setting a third parameter $Cr0_a$ representing the odorant concentration absorbed at a predetermined initial time $t_0$ by the walls of the first virtual pipe portion 2A and a fourth parameter $Cr0_b$ representing the odorant concentration absorbed at a predetermined initial time $t_0$ by the walls of the second virtual pipe portion 2B.

As an alternative, the method comprises the receiving, i.e. setting (and storing) a fifth parameter $Cr0_{a\text{-}b}$ representing the odorant concentration absorbed at a predetermined initial time $t_0$ by the walls of the first and second virtual pipe portion 2A, 2B.

It goes without saying that the fifth parameter is alternative to the second and third ones; it is advantageous where, in the model, there is no distinction between the first and second virtual pipe portion 2A, 2B.

The method comprises a step of preparing (storing) a first mathematical function comprising a first calculation parameter $Ca0_i$ and a second calculation parameter $Cr0_i$.

The method comprises a step of making a first calculation relating to the first virtual portion 2A, by means of the first mathematical function, at a predetermined first time instant $t_1$ following the predetermined initial time $t_0$, to calculate a first concentration $Ca_a(t1)$ at the predetermined first time instant $t_1$ of the odorant defining the mobile portion of the first virtual portion 2A of the pipe 2.

The first calculation parameter $Ca0_i$ is set equal to the first parameter $Ca0_a$.

The second calculation parameter $Cr0_i$ is set equal to the third or fifth parameter $Cr0_a$, $Cr0_{a\text{-}b}$.

The method comprises a step of making a first calculation relating to the second virtual portion 2B, by means of said first mathematical function, at the predetermined first time instant $t_1$, to calculate a first concentration $Ca_b(t1)$ at the predetermined first time instant $t_1$ of the odorant defining the mobile portion of the second virtual portion 2B.

In said first calculation relating to the second portion 2B the first calculation parameter $Ca0_i$ is set equal to the second parameter $Ca0_b$.

Moreover, in said first calculation relating to the second portion 2B, the second calculation parameter $Cr0_i$ is set equal to the fourth or fifth parameter $Cr0_b$, $Cr0_{a\text{-}b}$.

The method comprises a following step of calculating, at a second time instant t2 following the first time instant $t_1$, by means of said first mathematical formula, a second concentration $Ca_b(t2)$ at the predetermined second time instant $t_2$ of the odorant of the mobile portion inside the second virtual portion 2B of the pipe 2, in which in the first mathematical formula said first calculation parameter $Ca0_i$ was set equal to the value calculated at a predetermined first time instant $t_1$ of the first concentration $Ca_a(t1)$ of the odorant defining the mobile portion of the first virtual portion 2A of the pipe 2.

Advantageously, calculating at a second time instant t2 following the first time instant $t_1$, by means of said first mathematical formula, a second concentration $Ca_b(t2)$ at the predetermined second time instant $t_2$ of the odorant of the mobile portion inside the second virtual portion 2B of the pipe, allows to evaluate if the quantity of odorant in output from the second virtual portion is sufficient to guarantee safety of the gas user.

So, the method defines a simulation model of the odorant concentration in the gas in output from the pipe 2.

The quality of such model is clear for example in the graph of figure 3, where the solid line shows the evolution of the odorant concentration measured in output from the pipe 2 while the dashed line shows the odorant concentration simulated by the model.

The graph of figure 3 was obtained by means of an experimental test; advantageously the Applicant has tested, with practical tests, that the method object of the present invention allows to estimate with optimal performance (i.e. high precision) the quantity measured in the mobile phase in the pipe 2.

Advantageously, such method allows to reproduce satisfactorily the concentration of odorant in output.

So, thanks to such method it is possible to guarantee the presence of the optimal quantity of odorant in output both to guarantee the user safety and to avoid odorant waste.

In a preferred embodiment, the method comprises a step of preparing (storing) a second mathematical function comprising the first calculation parameter $Ca0_i$ and the second calculation parameter $Cr0_i$.

In such embodiment, the method comprises a step of calculating, at the predetermined first time instant $t_1$, by means of the second mathematical function, a concentration $Cr0_b(t1)$ of the odorant absorbed at the first time instant $t_1$ in the walls of the second virtual pipe portion 2B.

In the step of calculating, at the second time instant t2 following the first time instant $t_1$, by means of said first mathematical formula, a second concentration $Ca_b(t2)$ at the predetermined second time instant $t_2$ of the odorant of the mobile portion inside the second virtual portion 2B of the pipe 2, said second calculation parameter $Cr0_i$ is set equal to the calculated concentration $Cr0_b(t1)$ of the odorant absorbed at the first time instant $t_1$ in the walls of the second virtual portion 2B of the pipe.

In an embodiment, the second formula is obtained from the first formula on the basis of a mass balancing equation, in a virtual portion 2A, 2B, of the mass of odorant, absorbed or mobile respectively, between two consecutive time instants, preferably the predetermined initial time $t_0$ and the predetermined first time instant $t_1$.

In an example, the mass balancing equation, for a predetermined virtual pipe portion 2A, 2B, is of the type:

$$ma0 + mr0 = ma(t1) + mr(t1)$$

wherein

- ma0 represents the gaseous odorant mass in the predetermined virtual pipe portion 2A, 2B at the predetermined initial time $t_0$;
- mr0 represents the odorant mass absorbed by the walls of the predetermined virtual pipe portion 2A, 2B at the predetermined initial time $t_0$;
- $ma(t_1)$ represents the gaseous odorant mass in the predetermined virtual pipe portion 2A, 2B at the predetermined first time instant $(t_1)$;
- $mr(t_1)$ represents the odorant mass absorbed by the walls of the predetermined virtual pipe portion 2A, 2B at the predetermined first time instant $t_1$.

According to an aspect, the total odorant mass contained in a portion is supposed non to vary during the time interval between t0 and t1.

It simply occurs a movement of the odorant between the mobile portion and the fixed portion, and vice versa.

In a preferred embodiment, the first mathematical function comprises:

- a first mathematical relationship (i.e. portion of function) representing a transfer of the odorant from the mobile portion to the fixed portion;
- and a second mathematical relationship (i.e. portion of function) representing a transfer of the odorant from the fixed portion to the mobile portion.

According to an aspect, the first relationship and the second relationship comprise respective time constants $K1_a$, $K2_a$ and $K1_b$, $K2_b$ expressing a transfer speed of the odorant from the mobile portion to the fixed portion or vice versa.

Advantageously, the time constants $K1_a$, $K2_a$ and $K1_b$, $K2_b$ allow a reversible reaction to be simulated by considering respectively an adsorption from the mobile portion to the fixed portion and a desorption of the odorant from the fixed portion to the mobile portion.

In an embodiment, the first mathematical function comprises a relationship of the type:

$$Ca_i (t)=(k1_i*Ca0_i-K2*Cr0_i)* y(t)$$

wherein:

- $Ca_i(t)$ represents the odorant concentration defining the mobile portion at the time instant t in the i-th virtual portion of the pipe, first or second 2A, 2B;
- $K1_i$ is a numerical constant belonging to real numbers relating to the i-th virtual portion of the pipe, first or second 2A, 2B;
- $K2_i$ is a numerical constant belonging to real numbers;
- y(t) is a variable function in the time domain.

Advantageously, the first mathematical function allows to calculate the evolution of the odorant concentration in the gas over time.

The function y(t) is preferably a function decreasing over time.

In a preferred embodiment, the first mathematical function comprises a relationship of the type:

$$Ca_i (t)=(K1_i *Ca0_i- K2_a *Cr0_i)*u\hat{}(- K3_i * t) + K4_i$$

wherein:

- $Ca_i(t)$ represents the odorant concentration defining the mobile portion at the time instant t in the i-th virtual portion of the pipe, first or second 2A, 2B;
- $K1_i$ is a numerical constant belonging to real numbers relating to the i-th virtual portion of the pipe, first or second 2A,

2B;

- $K2_i$ is a numerical constant belonging to real numbers relating to the i-th portion, first or second 2A, 2B;
- $K3_i$ is a numerical constant belonging to real numbers relating to the i-th virtual portion of the pipe, first or second 2A, 2B, dependent on $K1_i$ and $K2_i$;
- $K4_i$ is a numerical constant belonging to real numbers relating to the i-th virtual portion of the pipe, dependent on $Cr0_i$ and $Ca0_i$, first or second 2A, 2B;
- t is the time passed from the predetermined initial time $t_0$;
- u is a numerical constant belonging to real numbers satisfying the following conditions: $0<u<1$ or $u>1$.

Preferably, u is between 1.5 and 3.

More preferably, u is between e -0.5 and e + 0.5, more preferably u is between e -0.25 and e + 0.25, wherein e is the Euler's number.

In an embodiment, the method comprises the step of setting (storing) a first volumetric calculation parameter relating to the volume $Vm_i$ of at least a first virtual portion 2A, 2B of the pipe 2 occupied by the odorant mobile portion.

In an embodiment, the method comprises a step of setting (and storing) a second volumetric calculation parameter relating to the volume $Vf_i$ of odorant absorbed by the walls of the second virtual pipe portion 2B, and in which the first mathematical function comprises also the first and second volumetric calculation parameter.

The volume $Vf_i$ of odorant is to be intended proportional to an inner surface of the pipe.

According to an aspect, the volume $Vf_i$ is linked to a value of the diameter of the pipe.

According to an aspect, the second mathematical function can comprise also the first and second volumetric calculation parameter.

In an embodiment, the first mathematical function comprises a relationship of the type:

$$Ca_i\,(t)=(K1_i\,{*}Ca0_i - K2_i\,{*}Cr0_i){*}u\verb|^|(-\,K3_i\,{*}\,t) + K5_i\,{*}\,K4_i$$

wherein:

- $Ca_i(t)$ represents the odorant concentration defining the mobile portion at the time instant t in the i-th portion, first or second 2A; 2B;
- $K1_i$ is a numerical variable depending on the first and second volumetric calculation parameter relating to the i-th virtual portion of the pipe, first or second 2A, 2B;
- $K2_i$ is a numerical variable depending on the first and second volumetric calculation parameter relating to the i-th virtual portion of the pipe, first or second 2A, 2B;
- $K3_i$ is a numerical variable depending on the first and second volumetric calculation parameter relating to the i-th virtual portion of the pipe, first or second 2A, 2B, on $K1_i$ and $K2_i$;
- $K4_i$ is a numerical variable depending on Ca0i, the first and second volumetric calculation parameter relating to the i-th virtual portion of the pipe, first or second 2A, 2B;
- $K5_i$ is a numerical variable depending on Cr0i, the first and second volumetric calculation parameter relating to the i-th virtual portion of the pipe, first or second 2A, 2B and on $K1_i$ and $K2_i$;
- t is the time passed from the predetermined initial time $t_0$;
- u is a numerical constant belonging to real numbers satisfying the following conditions: $0<u<1$ or $u>1$.

In a preferred embodiment, the first mathematical function relating to an i-th virtual portion of the pipe, first or second 2A, 2B, is the following:

$$Cai(t) = Ca0i \cdot \frac{(k1i - k2i \cdot Mi) \cdot Vfi \cdot e^{-\left(k1i + k2i \cdot \frac{Vmi}{Vfi}\right) \cdot t} + k2i \cdot (Vmi + Mi \cdot Vfi)}{k1i \cdot Vfi + k2i \cdot Vmi}$$

wherein:

- $Ca_i(t)$ represents the odorant concentration defining the mobile portion at the time instant t in the i-th portion, first or second 2A; 2B;
- $K1_i$ and $K2_i$ are two numerical constants belonging to real numbers relating to the i-th virtual portion of the pipe, first or second 2A, 2B;
- $M_i = Cr0_i/Ca0_i$, i.e. the ratio between the concentration at the predetermined instant $t_0$ of odorant in the fixed portion and the concentration at the predetermined instant $t_0$ of odorant in the mobile portion relating to the i-th virtual portion, first or second 2A, 2B;
- $Vm_i$ represents said first volumetric calculation parameter relating to the i-th virtual portion, first or second 2A, 2B;
- $Vf_i$ represents said second volumetric calculation parameter relating to the i-th virtual portion, first or second 2A, 2B;
- e represents the Euler's number;
- t represents the time instant object of the calculation.

According to an aspect, $K1_i$ is preferably between 0 and 5, more preferably between 0.00001 and 1.
According to an aspect, $K2_i$ is preferably between 0 and 5, more preferably between 0.00001 and 1.
In a preferred embodiment, the first mathematical relationship representing the transfer of the odorant from the mobile portion to the fixed portion is individuated by the following portion of function:

$$Ca0i \cdot \frac{k1i \cdot Vfi \cdot e^{-\left(k1i+k2i \cdot \frac{Vmi}{Vfi}\right) \cdot t}}{k1i \cdot Vfi + k2i \cdot Vmi}$$

In a preferred embodiment, the second mathematical relationship representing the transfer of odorant from the (fixed) portion to the (mobile) portion is individuated by the following portion of function:

$$Ca0i \cdot \frac{(-k2i \cdot Mi) \cdot Vfi \cdot e^{-\left(k1i+k2i \cdot \frac{Vmi}{Vfi}\right) \cdot t}}{k1i \cdot Vfi + k2i \cdot Vmi}$$

In a preferred embodiment, the second mathematical function relating to an i-th virtual portion of the pipe, first or second 2A, 2B, is the following:

$$Cr_i\,(t) = Ca0_i * M_i + (Ca0_i - Ca_i(t)) * Vm_i / Vf_i$$

wherein:

- $Cr_i(t)$ represents the odorant concentration absorbed at the time instant t by the walls of the i-th portion, first or second 2A; 2B;
- $Ca0_i$ represents the concentration at the predetermined instant $t_0$ of odorant in the mobile portion of the i-th virtual portion of the pipe;
- $Ca_i(t)$ represents the concentration at the time t of odorant in the mobile portion of the i-th virtual portion of the pipe, calculated by means of said first formula;
- $M_i = Cr0_i/Ca0_i$ is the ratio between the concentration at the predetermined instant $t_0$ of odorant in the fixed portion and the concentration at the predetermined instant $t_0$ of odorant in the mobile portion relating to the i-th virtual portion, first or second 2A, 2B;
- $Vm_i$ represents said first volumetric calculation parameter relating to the i-th virtual portion, first or second 2A, 2B;
- $Vf_i$ represents said second volumetric calculation parameter relating to the i-th virtual portion, first or second 2A, 2B;
- t represents the time instant object of the calculation.

According to an aspect, the step of setting, at a predetermined initial time $t_0$, a first parameter $Ca0_a$ representing the odorant concentration of the mobile portion of the first virtual portion 2A of the pipe 2 comprises a step of receiving a signal containing a piece of information relating to a flow of odorant introduced in the first virtual portion A2 of the pipe.
In an embodiment, said first mathematical formula comprises at least another calculation parameter dependent on the gas flow rate in the virtual portion 2A, 2B of the pipe 2.
According to an aspect, $K1_i$ depends on the gas flow rate in at least a virtual portion 2A, 2B of the pipe 2.
When the flow rate increases, there is an increase in the turbulence of the gas flow and an increase in the adsorbed odorant quantity; in output there is a lower odorant concentration, since the adsorption phenomenon prevails over the releasing phenomenon.
Vice versa, in case the flow rate decreases the releasing phenomenon prevails over the adsorption, in output there is an increase in the odorant concentration.
Object of the description is a computer product schematized in figure 1, comprising operating instructions which, when loaded on a processor 5, allow the processor 5 to execute the steps of the method according to any one of the preceding claims.

Object of the description is an apparatus 3 for estimating the concentration of an odorant in a gas flowing in a predetermined volume V of a pipe 2.

Such apparatus 3 shown in figure 1 comprises a processor 5 having, loaded in the memory, the computer product according to the preceding claim.

Advantageously, the estimation apparatus 3 allows to estimate the odorant concentration in output from the pipe.

Advantageously, estimating the odorant concentration in output from the pipe 2 allows to evaluate if it is guaranteed or not the safety for the final user the gas is supplied to.

Object of the description, shown in figure 2, is a gas distribution system comprising:

- a pipe 2 for the gas distribution;
- the apparatus 3 for estimating the gas concentration according to the preceding claim;
- and at least an odorant injector 4, configured to release the odorant inside the pipe 2.

Preferably, the system comprises at least a sensor 6 configured to detect a parameter relating to an odorant concentration in at least a first portion 2A' of the pipe 2, corresponding to the first virtual portion 2A, and wherein the processor 5 is connected to said sensor 6 to receive information about the odorant concentration in the first portion 2A' of the pipe 2 and to compare said received information with said estimated concentration $Ca_a(t1)$ of the odorant defining the mobile portion of the first virtual portion 2A.

The sensor 6 is preferably arranged at the odorant injector 4.

According to another aspect of the method, the method comprises a step of providing an adjustment signal of said odorant injector 4, configured to release odorant inside the pipe 2 (real), in particular the first portion 2A', said adjustment signal being function of the second concentration ($Ca_b(t2)$) at the predetermined second time instant ($t_2$) of the odorant of the mobile portion inside the second virtual portion 2B' of the pipe 2.

Moreover, preferably, the system comprises another sensor 7.

The other sensor 7 is preferably arranged at the output, i.e. at the connection point of the pipe 2.

According to another aspect, the other sensor 7 is configured to detect a parameter relating to an odorant concentration in at least a second portion 2B' of the pipe 2.

Such other second sensor 7 is preferably connected to the processor 5.

According to an aspect, the processor 5 is configured to receive the second parameter ($Ca0_b$) by means of a signal from the sensor 7 or by means of a user interface.

According to another aspect, the processor 5 is configured to compare said information received by the second sensor 7, and to compare it with the odorant estimated concentration, with the aim of confirming the quality of the estimation obtained with the method object of the present invention.

It is to be observed that, in figure 2, the input of the gas is in the portion 2A' of the pipe 2, while the output of the gas is in the portion 2B' of the pipe 2.

## Claims

1. A method, implemented by means of a processor (5), for estimating the concentration of an odorant in a gas flowing in a pipe (2), the method being **characterized in that** it comprises the following steps:

   - subdividing the pipe (2) in a plurality of virtual portions (2A, 2B) of the pipe (2), said plurality of virtual portions (2A, 2B) comprising at least a first virtual portion (2A) and a second virtual portion (2B) of the pipe (2);
   - receiving a first parameter ($Ca0_a$) representing, at a predetermined initial time ($t_0$), the odorant concentration in gaseous phase defining a mobile portion inside the first virtual portion (2A);
   - receiving a second parameter ($Ca0_b$) representing, at a predetermined initial time ($t_0$), the odorant concentration in gaseous phase defining a mobile portion inside the second virtual portion (2B);
   - receiving a third parameter ($Cr0_a$) representing the odorant concentration absorbed at a predetermined initial time ($t_0$) by the walls of the first virtual pipe portion (2A) and a fourth parameter ($Cr0_b$) representing the odorant concentration absorbed at a predetermined initial time ($t_0$) by the walls of the second virtual pipe portion (2B), or as an alternative, receiving a fifth parameter ($Cr0_{a\text{-}b}$) representing the odorant concentration absorbed at a predetermined initial time ($t_0$) by the walls of the first and second virtual pipe portion (2A, 2B);
   - preparing a first mathematical function comprising a first calculation parameter ($Ca0_i$) and a second calculation parameter ($Cr0_i$);
   - making a first calculation relating to the first virtual pipe portion (2A), by means of the first mathematical function, at a predetermined first time instant ($t_1$) following the predetermined initial time ($t_0$), to calculate a first concentration ($Ca_a(t1)$) at the predetermined first time instant ($t_1$) of the odorant defining the mobile portion of the first virtual portion (2A) of the pipe (2), said first calculation parameter ($Ca0_i$) being set equal to the first parameter ($Ca0_a$) and said second calculation parameter ($Cr0_i$) being set equal to the third or fifth parameter ($Cr0_a$, $Cr0_{a-b}$);
- making a first calculation relating to the second virtual portion (2B), by means of said first mathematical function, at the predetermined first time instant ($t_1$), to calculate a first concentration ($Ca_b$(t1)) at the predetermined first time instant ($t_1$) of the odorant defining the mobile portion of the second virtual portion (2B), wherein in said first calculation relating to the second portion (2B) said first calculation parameter ($Ca0_i$) is set equal to the second parameter ($Ca0_b$) and said second calculation parameter ($Cr0_i$) is set equal to the fourth or fifth parameter ($Cr0_b$, $Cr0_{a-b}$);
- calculating, at a second time instant (t2) following the first time instant ($t_1$), by means of said first mathematical formula, a second concentration ($Ca_b$(t2)) at the predetermined second time instant ($t_2$) of the odorant of the mobile portion inside the second virtual portion (2B) of the pipe (2), wherein in the first mathematical formula said first calculation parameter ($Ca0_i$) was set equal to the value calculated at a predetermined first time instant ($t_1$) of the first concentration ($Ca_a$(t1)) of the odorant defining the mobile portion of the first virtual portion (2A) of the pipe (2).

**2.** The method according to claim 1, comprising a step of receiving information about the odorant concentration in the first portion (2A) of the pipe (2) and of comparing said received information with said estimated concentration $Ca_a$(t1) of the odorant defining the mobile portion of the first virtual portion (2').

**3.** The method according to any one of the preceding claims, wherein said step of receiving a first parameter ($Ca0_a$) comprises the step of receiving a signal from a sensor (6) or by means of a user interface, said signal representing said first parameter ($Ca0_a$).

**4.** The method according to any one of the preceding claims, wherein said step of receiving a second parameter ($Ca0_b$) comprises a step of receiving a signal from another sensor (7) or by means of a user interface, said signal representing said second parameter ($Ca0_b$).

**5.** The method according to any one of the preceding claims, wherein said step of receiving a third parameter ($Cr0_a$) and a fourth parameter ($Cr0_b$) or of receiving a fifth parameter ($Cr0_{a-b}$) comprises a step of receiving information from a user interface, said information comprising said third parameter ($Cr0_a$), fourth parameter ($Cr0_b$) or fifth parameter ($Cr0_{a-b}$).

**6.** The method according to any one of the preceding claims, comprising a step of providing an adjustment signal of an odorant injector (4), configured to release odorant inside the pipe (2), said adjustment signal being function of the second concentration ($Ca_b$(t2)) at the predetermined second time instant ($t_2$) of the odorant of the mobile portion inside the second virtual portion (2B) of the pipe (2).

**7.** The method according to any one of the preceding claims, comprising the following steps:

- preparing a second mathematical function comprising the first calculation parameter ($Ca0_i$) and second calculation parameter ($Cr0_i$);
- calculating, at the predetermined first time instant ($t_1$), by means of the second mathematical function, a concentration $Cr0_b$(t1) of the odorant absorbed at the first time instant ($t_1$) in the walls of the second virtual pipe portion (2B),

and wherein in the step of calculating, at the second time instant (t2) following the first time instant ($t_1$), by means of said first mathematical formula a second concentration ($Ca_b$(t2)) at the predetermined second time instant ($t_2$) of the odorant of the mobile portion inside the second virtual portion (2B) of the pipe (2), said second calculation parameter ($Cr0_i$) is set equal to the calculated concentration $Cr0_b$(t1) of the odorant absorbed at the first time instant ($t_1$) in the walls of the second virtual portion (2B) of the pipe.

**8.** The method according to any one of the preceding claims, wherein the second formula is obtained from said first formula on the basis of a mass balancing equation, in a virtual portion (2A, 2B), of the mass of odorant, absorbed or mobile respectively, between two consecutive time instants, preferably the predetermined initial time ($t_0$) and the predetermined first time instant ($t_1$).

**9.** The method according to the preceding claim, wherein the mass balancing equation, for a predetermined virtual pipe portion (2A, 2B), is of the type:

$$ma0 + mr0 = ma(t1) + mr(t1)$$

wherein

- ma0 represents the gaseous odorant mass in the predetermined virtual pipe portion (2A, 2B) at the predetermined initial time ($t_0$);
- mr0 represents the odorant mass absorbed by the walls of the predetermined virtual pipe portion (2A, 2B) at the predetermined initial time ($t_0$);
- ma($t_1$) represents the gaseous odorant mass in the predetermined virtual pipe portion (2A, 2B) at the predetermined first time instant ($t_1$);
- mr($t_1$) represents the odorant mass absorbed by the walls of the predetermined virtual pipe portion (2A, 2B) at the predetermined first time instant ($t_1$).

**10.** The method according to any one of the preceding claims, wherein the first mathematical function comprises:

- a first mathematical relationship representing a transfer of the odorant from the mobile portion to the fixed portion;
- and a second mathematical relationship representing a transfer of the odorant from the fixed portion to the mobile portion.

**11.** The method according to any one of the preceding claims, wherein said first and the second relationships comprise respective time constants ($K1_a$, $K2_a$; $K1_b$, $K2_b$) expressing a transfer speed of the odorant from the mobile portion to the fixed portion or vice versa.

**12.** The method according to any one of the preceding claims 1 to 11, wherein said first mathematical function comprises a relationship of the type:

$$Ca_i\,(t)=(k1_i{*}Ca0_i-K2{*}Cr0_i){*}\ y(t)$$

wherein:

- $Ca_i(t)$ represents the odorant concentration defining the mobile portion at the time instant t in the i-th virtual portion of the pipe, first or second (2A, 2B);
- $K1_i$ is a numerical constant belonging to real numbers relating to the i-th virtual portion of the pipe, first or second (2A; 2B);
- $K2_i$ is a numerical constant belonging to real numbers;
- y(t) is a variable function in the time domain.

**13.** The method according to the preceding claim, wherein y(t) is a function decreasing over time.

**14.** The method according to any one of the preceding claims 1 to 13, wherein said first mathematical function comprises a relationship of the type:

$$Ca_i\,(t)=(K1_i\ {*}Ca0_i-\ K2_a\ {*}Cr0_i){*}u\hat{}(-\ K3_i\ {*}\ t)+K4_i$$

wherein:

- $Ca_i(t)$ represents the odorant concentration defining the mobile portion at the time instant t in the i-th virtual portion of the pipe, first or second (2A; 2B);
- $K1_i$ is a numerical constant belonging to real numbers relating to the i-th virtual portion of the pipe, first or second (2A; 2B);
- $K2_i$ is a numerical constant belonging to real numbers relating to the i-th portion, first or second (2A, 2B);
- $K3_i$ is a numerical constant belonging to real numbers relating to the i-th virtual portion of the pipe, first or second (2A; 2B), dependent on $K1_i$ and $K2_i$;

- $K4_i$ is a numerical constant belonging to real numbers relating to the i-th virtual portion of the pipe, dependent on Cr0i and Ca0i, first or second (2A; 2B);
- t is the time passed from the predetermined initial time $t_0$;
- u is a numerical constant belonging to real numbers satisfying the following conditions: $0<u<1$ or $u>1$.

**15.** The method according to the preceding claim, wherein u is between 1.5 and 3.

**16.** The method according to the preceding claim, wherein u is between e - 0.5 and e + 0.5, more preferably u is between e - 0.25 and e + 0.25, wherein e is the Euler's number.

**17.** The method according to any one of the preceding claims, comprising the steps of:

- setting a first volumetric calculation parameter relating to the volume ($Vm_i$) of at least a first virtual portion (2A, 2B) of the pipe (2) occupied by the odorant mobile portion;
- setting a second volumetric calculation parameter relating to the volume ($Vf_i$) of odorant absorbed by the walls of the second virtual pipe portion (2B), and wherein the first mathematical function comprises also the first and second volumetric calculation parameter.

**18.** The method according to claim 17 and claim 7, wherein the second mathematical function comprises also the first and second volumetric calculation parameter.

**19.** The method according to any one of the preceding claims and claim 17 or 18, wherein said first mathematical function comprises a relationship of the type:

$$Ca_i\,(t)=(K1_i\,{*}Ca0_i-K2_i\,{*}Cr0_i){*}u\text{^}(-K3_i\,{*}\,t)+K5_i\,{*}\,K4_i$$

wherein:

- $Ca_i(t)$ represents the odorant concentration defining the mobile portion at the time instant t in the i-th portion, first or second (2A; 2B);
- $K1_i$ is a numerical variable depending on the first and second volumetric calculation parameter relating to the i-th virtual portion of the pipe, first or second (2A; 2B);
- $K2_i$ is a numerical variable depending on the first and second volumetric calculation parameter relating to the i-th virtual portion of the pipe, first or second (2A; 2B);
- $K3_i$ is a numerical variable depending on the first and second volumetric calculation parameter relating to the i-th virtual portion of the pipe, first or second (2A; 2B), dependent on $K1_i$ and $K2_i$;
- $K4_i$ is a numerical variable depending on Ca0i, the first and second volumetric calculation parameter relating to the i-th virtual portion of the pipe, first or second (2A; 2B);
- $K5_i$ is a numerical variable depending on Cr0i, the first and second volumetric calculation parameter relating to the i-th virtual portion of the pipe, first or second (2A; 2B) and dependent on $K1_i$ and $K2_i$;
- t is the time passed from the predetermined initial time $t_0$;
- u is a numerical constant belonging to real numbers satisfying the following conditions: $0<u<1$ or $u>1$.

**20.** The method according to one of the claims 17 to 19, wherein said first mathematical function relating to an i-th virtual portion of the pipe, first or second (2A; 2B), is the following:

$$Cai(t) = Ca0i \cdot \frac{(k1i - k2i \cdot Mi) \cdot Vfi \cdot e^{-\left(k1i + k2i \cdot \frac{Vmi}{Vfi}\right) \cdot t} + k2i \cdot (Vmi + Mi \cdot Vfi)}{k1i \cdot Vfi + k2i \cdot Vmi}$$

wherein:

- $Ca_i(t)$ represents the odorant concentration defining the mobile portion at the time instant t in the i-th portion, first or second (2A; 2B);
- $K1_i$ and $K2_i$ are two numerical constants belonging to real numbers relating to the i-th virtual portion of the pipe, first or second (2A; 2B);
- $M_i$ - $Cr0_i/Ca0_i$, i.e. the ratio between the concentration at the predetermined instant $t_0$ of odorant in the fixed portion and the concentration at the predetermined instant $t_0$ of odorant in the mobile portion relating to the i-th virtual portion, first or second (2A; 2B);
- $Vm_i$ represents said first volumetric calculation parameter relating to the i-th virtual portion, first or second (2A; 2B);
- $Vf_i$ represents said second volumetric calculation parameter relating to the i-th virtual portion, first or second (2A; 2B);
- e represents the Euler's number;
- t represents the time instant object of the calculation.

**21.** The method according to any one of the preceding claims and to claims 7 and 17, wherein said second mathematical function relating to an i-th virtual portion of the pipe, first or second (2A; 2B), is the following:

$$Cr_i (t)= Ca0_i * M_i + (Ca0_i - Ca_i(t)) * Vm_i / Vf_i$$

wherein:

- $Cr_i(t)$ represents the odorant concentration absorbed at the time instant t by the walls of the i-th portion, first or second (2A; 2B);
- $Ca0_i$ represents the concentration at the predetermined instant $t_0$ of odorant in the mobile portion of the i-th virtual portion of the pipe;
- $Ca_i(t)$ represents the concentration at the time t of odorant in the mobile portion of the i-th virtual portion of the pipe, calculated by means of said first formula;
- $M_i = Cr0_i/Ca0_i$ is the ratio between the concentration at the predetermined instant $t_0$ of odorant in the fixed portion and the concentration at the predetermined instant $t_0$ of odorant in the mobile portion relating to the i-th virtual portion, first or second (2A; 2B);
- $Vm_i$ represents said first volumetric calculation parameter relating to the i-th virtual portion, first or second (2A; 2B);
- $Vf_i$ represents said second volumetric calculation parameter relating to the i-th virtual portion, first or second (2A; 2B);
- t represents the time instant object of the calculation.

**22.** The method according to any one of the preceding claims, wherein the step of setting, at a predetermined initial time ($t_0$), a first parameter ($Ca0_a$) representing the odorant concentration of the mobile portion of the first portion (2A) of the pipe (2) comprises a step of receiving a signal containing a piece of information relating to a flow of odorant introduced in the first portion (2A) of the pipe.

**23.** The method according to any one of the preceding claims, wherein said first mathematical formula comprises at least another calculation parameter depending on the gas flow rate in the virtual portion (2A, 2B) of the pipe.

**24.** The method according to any one of the preceding claims and claim 19, wherein $K1_i$ depends on the gas flow rate in at least a virtual portion (2A, 2B) of the pipe.

**25.** A computer product comprising operating instructions which, when loaded on a processor (5), allow the processor (5) to execute the steps of the method according to any one of the preceding claims.

**26.** An apparatus (3) for estimating the concentration of an odorant in a gas flowing in a predetermined volume (V) of a pipe (2), comprising a processor (5) having, loaded in the memory, the computer product according to the preceding claim.

**27.** A gas distribution system, comprising:

- a pipe (2) for the gas distribution;
- the apparatus (3) for estimating the gas concentration according to the preceding claim;
- and at least an odorant injector (4), configured to release the odorant inside the pipe (2).

**28.** The system according to the preceding claim, comprising at least a sensor (6) configured to detect a parameter relating to an odorant concentration in at least a first portion (2A') of the pipe (2), corresponding to the first virtual portion (2A), and wherein the processor (5) is connected to said sensor (6) to receive information about the odorant concentration in the first portion (2A') of the pipe (2) and to compare said received information with said estimated concentration ($Ca_a(t1)$) of the odorant defining the mobile portion of the first virtual portion (2A) of the pipe (2).

Fig.1

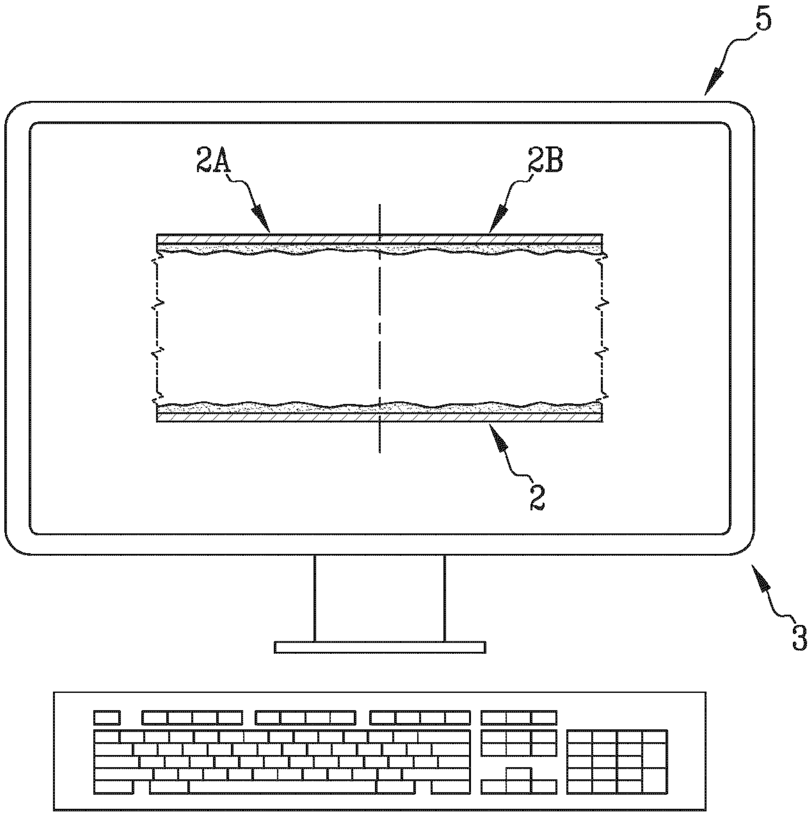


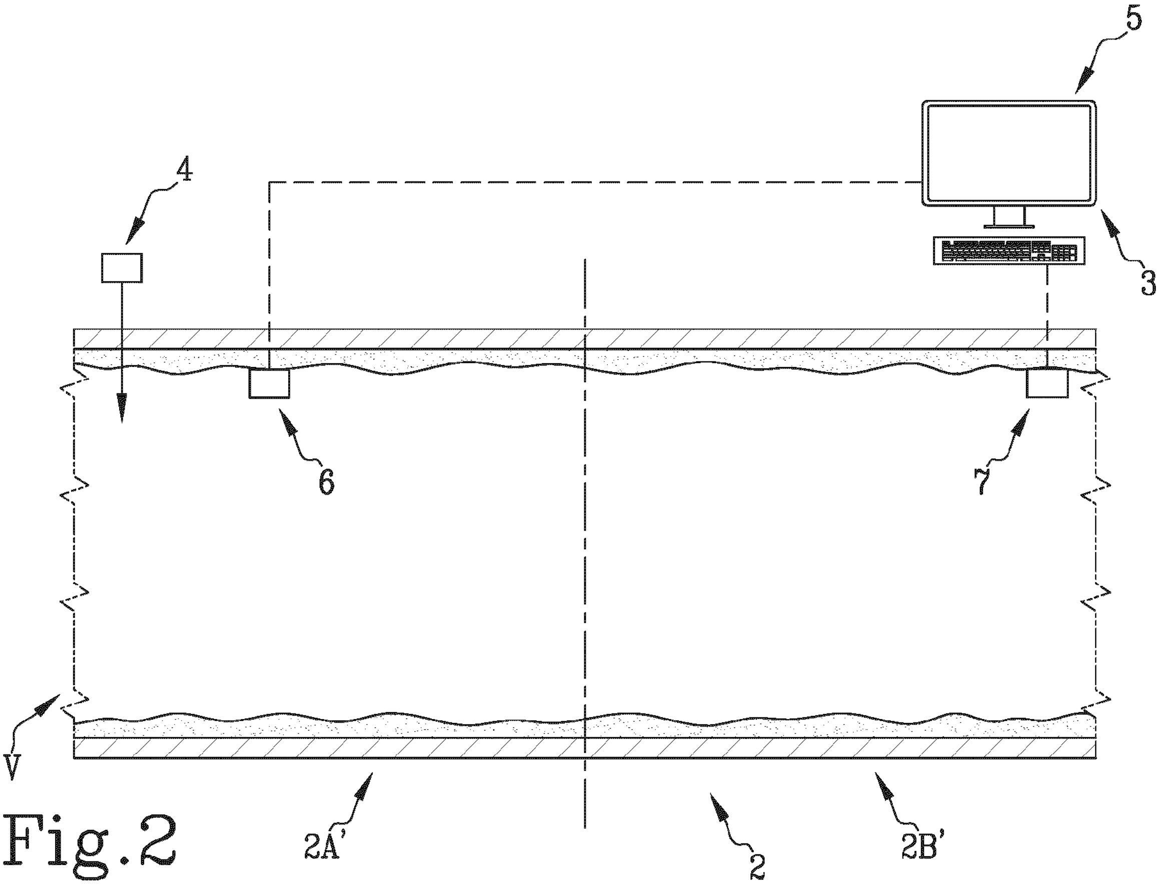


Fig.2

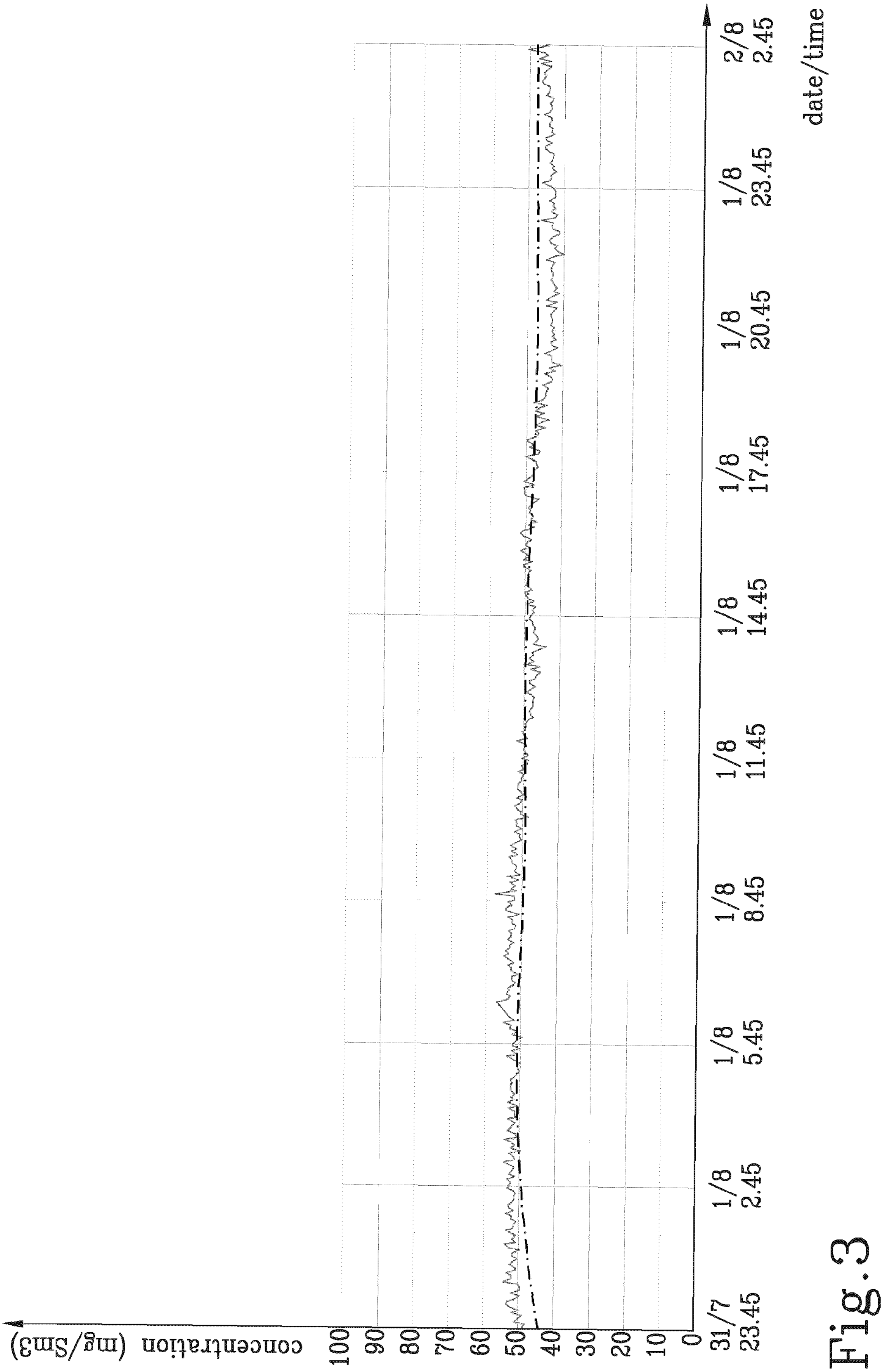
concentration (mg/Sm3)
100
90
80
70
60
50
40
30
20
10
0
31/7 23.45
1/8 2.45
1/8 5.45
1/8 8.45
1/8 11.45
1/8 14.45
1/8 17.45
1/8 20.45
1/8 23.45
2/8 2.45
date/time

Fig.3

Europäisches Patentamt
European Patent Office
Office européen des brevets

# EUROPEAN SEARCH REPORT

Application Number
EP 25 18 2758

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br>A | DEYMI-DASHTEBAYAZ MAHDI ET AL: "Experimental and numerical investigation of odorant dispersion in natural-gas pipelines", THE EUROPEAN PHYSICAL JOURNAL PLUS, SPRINGER BERLIN HEIDELBERG, BERLIN/HEIDELBERG, vol. 134, no. 6, 26 June 2019 (2019-06-26), pages 1-13, XP036821424, DOI: 10.1140/EPJP/I2019-12666-2<br>* abstract *<br>* page 2, paragraph 2-4 *<br>* page 11; table 3 *<br>----- | 1-5,7-27<br><br><br><br><br><br><br><br><br><br>6,28 | INV.<br>G16C60/00 |
| X<br><br><br>A | CN 117 272 235 A (BEIJING BAOLONG HONGRUI TECH CO LTD; CHONGQING HONGBAO TECH CO LTD) 22 December 2023 (2023-12-22)<br>* claims 1-6 *<br>----- | 1-5,7-27<br><br><br>6,28 | |
| A | GALASSI ROSSANA ET AL: "Odorant Monitoring in Natural Gas Pipelines Using Ultraviolet-Visible Spectroscopy", APPLIED SPECTROSCOPY.<br>,<br>vol. 75, no. 2<br>8 October 2020 (2020-10-08), pages 168-177, XP055947936,<br>US<br>ISSN: 0003-7028, DOI: 10.1177/0003702820960737<br>Retrieved from the Internet:<br>URL:http://journals.sagepub.com/doi/full-xml/10.1177/0003702820960737<br>* the whole document *<br>----- | 1-28 | TECHNICAL FIELDS SEARCHED (IPC)<br><br>G16C |
| A | US 3 686 930 A (KNIEBES DUANE V ET AL) 29 August 1972 (1972-08-29)<br>* the whole document *<br>----- | 1-28 | |

The present search report has been drawn up for all claims

1

| Place of search | Date of completion of the search | Examiner |
|---|---|---|
| The Hague | 17 October 2025 | Nurmi, Jussi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 25 18 2758

This annex lists the patent family members relating to the patent documents cited in the above-mentioned European search report. The members are as contained in the European Patent Office EDP file on
The European Patent Office is in no way liable for these particulars which are merely given for the purpose of information.

17-10-2025

| Patent document cited in search report | Publication date | Patent family member(s) | Publication date |
|---|---|---|---|
| CN 117272235 A | 22-12-2023 | NONE | |
| US 3686930 A | 29-08-1972 | CA 928523 A | 19-06-1973 |
| | | US 3686930 A | 29-08-1972 |

EPO FORM P0459

For more details about this annex : see Official Journal of the European Patent Office, No. 12/82